# EUROPEAN PATENT APPLICATION

(11) **EP 3 425 057 A1**
(43) Date of publication of application: **09.01.2019**
(21) Application number: 17760140.8
(22) Date of filing: 02.03.2017
(51) Int. Cl.: C12Q 1/68, C07K 16/00, C12N 5/20, C12N 15/09

(54) **METHOD FOR DETERMINING NUCLEIC ACID SEQUENCE OF TARGET GENE**

(30) Priority: 02.03.2016 US 201662302196 P
(71) Applicant: Kyushu University, National University Corporation, Fukuoka-shi, Fukuoka 812-8581 (JP)
(72) Inventor: OHKAWA Yasuyuki, Fukuoka-shi Fukuoka 812-8581 (JP); MAEHARA Kazumitsu, Fukuoka-shi Fukuoka 812-8581 (JP); KIMURA Hiroshi, Tokyo 152-8550 (JP); SATO Yuko, Tokyo 152-8550 (JP)
(74) Representative: EP&C
(86) International application number: PCT/JP2017/008320
(87) International publication number: WO 2017/150677

(57) **Abstract**

A method for determining a nucleic acid sequence of a target gene expressed in a subject cell, the method including: comprehensively determining mRNA nucleic acid sequences in the subject cell, and identifying a nucleic acid sequence having a nucleic acid sequence of a portion of the target gene, from among the determined mRNA nucleic acid sequences, in which the identified nucleic acid sequence is a nucleic acid sequence of the target gene.

SEQUENCE LISTING

<110>  KYUSHU UNIVERSITY

<120>  METHOD FOR DETERMINING NUCLEIC ACID SEQUENCE OF TARGET GENE

<130>  PC-23420

<150>  US62/302196
<151>  2016-03-02

<160>  33

<170>  PatentIn version 3.5

<210>  1
<211>  21
<212>  DNA
<213>  Rattus norvegicus

<400>  1
aaagcatgtg tgtctgtgat g                                                21


<210>  2
<211>  21
<212>  DNA
<213>  Rattus norvegicus

<400>  2
tgaaatcctc gcaggaaact c                                                21


<210>  3
<211>  20
<212>  DNA
<213>  Rattus norvegicus

<400>  3
tgattgccac agccttcagt                                                  20


<210>  4
<211>  20
<212>  DNA
<213>  Rattus norvegicus

<400>  4
catgaaaacc agcctgtcct                                                  20


<210>  5
<211>  20
<212>  DNA
<213>  Rattus norvegicus

<400>  5
aaatagccct tgaccaggca                                                  20


<210>  6
<211>  19
<212>  DNA

<213>  Rattus norvegicus

<400>  6
gaaggtcttt ctcagggct                                                    19


<210>  7
<211>  18
<212>  DNA
<213>  Rattus norvegicus

<400>  7
gctcagctgt actcatgc                                                     18


<210>  8
<211>  21
<212>  DNA
<213>  Rattus norvegicus

<400>  8
ggttggttgt catcttactg t                                                 21


<210>  9
<211>  21
<212>  DNA
<213>  Rattus norvegicus

<400>  9
cttgtcttgt tggcttgaga t                                                 21


<210>  10
<211>  22
<212>  DNA
<213>  Rattus norvegicus

<400>  10
tgatgtctct gggatagaag tt                                                22


<210>  11
<211>  20
<212>  DNA
<213>  Rattus norvegicus

<400>  11
tgtgcccaga aactgtggag                                                   20


<210>  12
<211>  21
<212>  DNA
<213>  Rattus norvegicus

<400>  12
gccaagggaa tgcaatcctt g                                                 21


<210>  13
<211>  24
<212>  DNA

<213>  Rattus norvegicus

<400>  13
caaacaacag ccccatctgt ctat                                              24


<210>  14
<211>  23
<212>  DNA
<213>  Rattus norvegicus

<400>  14
agaacaacag ccccatctgt cta                                               23


<210>  15
<211>  20
<212>  DNA
<213>  Rattus norvegicus

<400>  15
caacccaagg ctacgccctc                                                   20


<210>  16
<211>  20
<212>  DNA
<213>  Rattus norvegicus

<400>  16
cagcccaagt ccactcccac                                                   20


<210>  17
<211>  30
<212>  DNA
<213>  Rattus norvegicus

<400>  17
accaactgta tctatcttcc caccatccac                                        30


<210>  18
<211>  20
<212>  DNA
<213>  Mus musculus

<400>  18
ccaaaacgac acccccatct                                                   20


<210>  19
<211>  22
<212>  DNA
<213>  Mus musculus

<400>  19
gtgtgtggag atacaactgg ct                                                22


<210>  20
<211>  21
<212>  DNA

<213>  Mus musculus

<400>  20
ccaaaacaac acccccatca g                                                21


<210>  21
<211>  23
<212>  DNA
<213>  Mus musculus

<400>  21
gtgtggaggt acaactggct cct                                              23


<210>  22
<211>  21
<212>  DNA
<213>  Mus musculus

<400>  22
ctacaacaac agccccatct g                                                21


<210>  23
<211>  21
<212>  DNA
<213>  Mus musculus

<400>  23
gccagcccaa gtcttcgcca t                                                21


<210>  24
<211>  24
<212>  DNA
<213>  Mus musculus

<400>  24
gtcagcccaa gtccactccc actc                                             24


<210>  25
<211>  24
<212>  DNA
<213>  Mus musculus

<400>  25
gtcagcccaa gtccactccc acac                                             24


<210>  26
<211>  24
<212>  DNA
<213>  Mus musculus

<400>  26
gccaacccaa ggctacaccc tcag                                             24


<210>  27
<211>  21
<212>  DNA

<213>  Mus musculus

<400>  27
gggctgatgc tgcaccaact g                                                21


<210>  28
<211>  1395
<212>  DNA
<213>  Rattus norvegicus

<400>  28
atgaaatgca ggtggatcat cctcttcttg atggcagtag ctacaggggt caactcagaa      60

gtccagctgc agcaatctgg gcctgagctt cagagacccg gggcctcagt caagttgtcg     120

tgcaaggctt ctggctatac ctttacagaa tactatatgt actgggtgaa gcagaggcct     180

aaacagggcc tggaattaat aggaaggatt gatcctgaag acggtagtac tgattatgtt     240

gagaagttca aaaacaaggc cacactgact gcagatacat cgtccaacac agcctacatg     300

caactcagca gcctgacatc tgaggacaca gcaacctatt tttgtgcggc gggaactagg     360

tggggccaag gagtcatggt cacagtctcc tcagcccaaa caacagcccc atctgtctat     420

ccactggctc ctggatgtgg tgatacaacc agctccacgg tgactctggg atgcctggtc     480

aagggctatt tccctgagcc agtcaccgtg acctggaact ctggagccct gtccagcgat     540

gtgcacacct ttccagctgt cctgcagtct gggctctaca ctctcaccag ctcagtgacc     600

tccagcacct ggcccagcca gaccgtcacc tgcaacgtag cccacccggc cagcagcacc     660

aaggtggaca agaaagttga gcgcagaaat ggcggcattg gacacaaatg ccctacatgc     720

cctacatgtc acaaatgccc agttcctgaa ctcttgggtg gaccatctgt cttcatcttc     780

ccgccaaagc ccaaggacat cctcttgatc tcccagaacg ccaaggtcac gtgtgtggtg     840

gtggatgtga gcgaggagga gccggacgtc cagttcagct ggtttgtgaa caacgtagaa     900

gtacacacag ctcagacaca accccgtgag gagcagtaca acagcacctt cagagtggtc     960

agtgccctcc ccatccagca ccaggactgg atgagcggca aggagttcaa atgcaaggtc    1020

aacaacaaag ccctcccaag ccccatcgag aaaaccatct caaaacccaa agggctagtc    1080

agaaaaccac aggtatacgt catgggtcca ccgacagagc agttgactga gcaaacggtc    1140

agtttgacct gcttgacctc aggcttcctc cctaacgaca tcggtgtgga gtggaccagc    1200

aacgggcata tagaaaagaa ctacaagaac accgagccag tgatggactc tgacggttct    1260

ttcttcatgt acagcaagct caatgtggaa aggagcaggt gggatagcag agcgcccttc    1320

gtctgctccg tggtccacga gggtctgcac aatcaccacg tggagaagag catctcccgg    1380

cctccgggta aatga                                                     1395


<210>  29
<211>  464

<212>  PRT
<213>  Rattus norvegicus

<400>  29

Met Lys Cys Arg Trp Ile Ile Leu Phe Leu Met Ala Val Ala Thr Gly
1               5                   10                  15

Val Asn Ser Glu Val Gln Leu Gln Gln Ser Gly Pro Glu Leu Gln Arg
            20                  25                  30

Pro Gly Ala Ser Val Lys Leu Ser Cys Lys Ala Ser Gly Tyr Thr Phe
        35                  40                  45

Thr Glu Tyr Tyr Met Tyr Trp Val Lys Gln Arg Pro Lys Gln Gly Leu
    50                  55                  60

Glu Leu Ile Gly Arg Ile Asp Pro Glu Asp Gly Ser Thr Asp Tyr Val
65                  70                  75                  80

Glu Lys Phe Lys Asn Lys Ala Thr Leu Thr Ala Asp Thr Ser Ser Asn
                85                  90                  95

Thr Ala Tyr Met Gln Leu Ser Ser Leu Thr Ser Glu Asp Thr Ala Thr
            100                 105                 110

Tyr Phe Cys Ala Ala Gly Thr Arg Trp Gly Gln Gly Val Met Val Thr
        115                 120                 125

Val Ser Ser Ala Gln Thr Thr Ala Pro Ser Val Tyr Pro Leu Ala Pro
    130                 135                 140

Gly Cys Gly Asp Thr Thr Ser Ser Thr Val Thr Leu Gly Cys Leu Val
145                 150                 155                 160

Lys Gly Tyr Phe Pro Glu Pro Val Thr Val Thr Trp Asn Ser Gly Ala
                165                 170                 175

Leu Ser Ser Asp Val His Thr Phe Pro Ala Val Leu Gln Ser Gly Leu
            180                 185                 190

Tyr Thr Leu Thr Ser Ser Val Thr Ser Ser Thr Trp Pro Ser Gln Thr
        195                 200                 205

Val Thr Cys Asn Val Ala His Pro Ala Ser Ser Thr Lys Val Asp Lys
    210                 215                 220

Lys Val Glu Arg Arg Asn Gly Gly Ile Gly His Lys Cys Pro Thr Cys
225                 230                 235                 240

Pro Thr Cys His Lys Cys Pro Val Pro Glu Leu Leu Gly Gly Pro Ser
                245                 250                 255

Val Phe Ile Phe Pro Pro Lys Pro Lys Asp Ile Leu Leu Ile Ser Gln
            260                 265                 270

Asn Ala Lys Val Thr Cys Val Val Val Asp Val Ser Glu Glu Glu Pro
        275                 280                 285

Asp Val Gln Phe Ser Trp Phe Val Asn Asn Val Glu Val His Thr Ala
    290                 295                 300

Gln Thr Gln Pro Arg Glu Glu Gln Tyr Asn Ser Thr Phe Arg Val Val
305                 310                 315                 320

Ser Ala Leu Pro Ile Gln His Gln Asp Trp Met Ser Gly Lys Glu Phe
                325                 330                 335

Lys Cys Lys Val Asn Asn Lys Ala Leu Pro Ser Pro Ile Glu Lys Thr
            340                 345                 350

Ile Ser Lys Pro Lys Gly Leu Val Arg Lys Pro Gln Val Tyr Val Met
        355                 360                 365

Gly Pro Pro Thr Glu Gln Leu Thr Glu Gln Thr Val Ser Leu Thr Cys
    370                 375                 380

Leu Thr Ser Gly Phe Leu Pro Asn Asp Ile Gly Val Glu Trp Thr Ser
385                 390                 395                 400

Asn Gly His Ile Glu Lys Asn Tyr Lys Asn Thr Glu Pro Val Met Asp
                405                 410                 415

Ser Asp Gly Ser Phe Phe Met Tyr Ser Lys Leu Asn Val Glu Arg Ser
            420                 425                 430

Arg Trp Asp Ser Arg Ala Pro Phe Val Cys Ser Val Val His Glu Gly
        435                 440                 445

Leu His Asn His His Val Glu Lys Ser Ile Ser Arg Pro Pro Gly Lys
    450                 455                 460

<210>  30
<211>  332
<212>  PRT
<213>  Rattus norvegicus

<400> 30

Gln Thr Thr Ala Pro Ser Val Tyr Pro Leu Ala Pro Gly Cys Gly Asp
1               5                   10                  15

Thr Thr Ser Ser Thr Val Thr Leu Gly Cys Leu Val Lys Gly Tyr Phe
            20                  25                  30

Pro Glu Pro Val Thr Val Thr Trp Asn Ser Gly Ala Leu Ser Ser Asp
        35                  40                  45

Val His Thr Phe Pro Ala Val Leu Gln Ser Gly Leu Tyr Thr Leu Thr
    50                  55                  60

Ser Ser Val Thr Ser Ser Thr Trp Pro Ser Gln Thr Val Thr Cys Asn
65                  70                  75                  80

Val Ala His Pro Ala Ser Ser Thr Lys Val Asp Lys Lys Val Glu Arg
                85                  90                  95

Arg Asn Gly Gly Ile Gly His Lys Cys Pro Thr Cys Pro Thr Cys His
            100                 105                 110

Lys Cys Pro Val Pro Glu Leu Leu Gly Gly Pro Ser Val Phe Ile Phe
        115                 120                 125

Pro Pro Lys Pro Lys Asp Ile Leu Leu Ile Ser Gln Asn Ala Lys Val
    130                 135                 140

Thr Cys Val Val Val Asp Val Ser Glu Glu Glu Pro Asp Val Gln Phe
145                 150                 155                 160

Ser Trp Phe Val Asn Asn Val Glu Val His Thr Ala Gln Thr Gln Pro
                165                 170                 175

Arg Glu Glu Gln Tyr Asn Ser Thr Phe Arg Val Val Ser Ala Leu Pro
            180                 185                 190

Ile Gln His Gln Asp Trp Met Ser Gly Lys Glu Phe Lys Cys Lys Val
        195                 200                 205

Asn Asn Lys Ala Leu Pro Ser Pro Ile Glu Lys Thr Ile Ser Lys Pro
    210                 215                 220

Lys Gly Leu Val Arg Lys Pro Gln Val Tyr Val Met Gly Pro Pro Thr
225                 230                 235                 240

Glu Gln Leu Thr Glu Gln Thr Val Ser Leu Thr Cys Leu Thr Ser Gly

245                 250                 255

Phe Leu Pro Asn Asp Ile Gly Val Glu Trp Thr Ser Asn Gly His Ile
            260                 265                 270

Glu Lys Asn Tyr Lys Asn Thr Glu Pro Val Met Asp Ser Asp Gly Ser
        275                 280                 285

Phe Phe Met Tyr Ser Lys Leu Asn Val Glu Arg Ser Arg Trp Asp Ser
    290                 295                 300

Arg Ala Pro Phe Val Cys Ser Val Val His Glu Gly Leu His Asn His
305                 310                 315                 320

His Val Glu Lys Ser Ile Ser Arg Pro Pro Gly Lys
                325                 330

<210>  31
<211>  705
<212>  DNA
<213>  Rattus norvegicus

<400>  31
atggtgttca aatttcagat ccttggactt ctgcttttct ggatttcagc ctctagaggg      60

gacatcgtgc tgactcagtc tccaaccacc ctgtctgtga ctccaggaga gacagtcagt     120

ctctcctgca gggctagcca tagtattggc acaaatctac actggtatca gcaaaaaaca     180

aatgagtctc caaggcttct catcaagtat tcttcccagt ccatctctgg gatcccctcc     240

aggttcagtg ccagtggatc agggacagat tttactctca acatcaacaa tgtggagttt     300

gatgatgtct caagttattt ttgtcaacag actcaaagct ggcccgacac gtttggagct     360

gggaccaagc tggaactgaa acgggctgat gctgcaccaa ctgtatctat cttcccacca     420

tccacggaac agttagcaac tggaggtgcc tcagtcgtgt gcctcatgaa caacttctat     480

cccagagaca tcagtgtcaa gtggaagatt gatggcactg aacgacgaga tggtgtcctg     540

gacagtgtta ctgatcagga cagcaaagac agcacgtaca gcatgagcag caccctctcg     600

ttgaccaagg ctgactatga aagtcataac ctctatacct gtgaggttgt tcataagaca     660

tcatcctcac ccgtcgtcaa gagcttcaac aggaatgagt gttag                     705

<210>  32
<211>  234
<212>  PRT
<213>  Rattus norvegicus

<400>  32

Met Val Phe Lys Phe Gln Ile Leu Gly Leu Leu Leu Phe Trp Ile Ser
1               5                   10                  15

Ala Ser Arg Gly Asp Ile Val Leu Thr Gln Ser Pro Thr Thr Leu Ser
            20                  25                  30

Val Thr Pro Gly Glu Thr Val Ser Leu Ser Cys Arg Ala Ser His Ser
        35                  40                  45

Ile Gly Thr Asn Leu His Trp Tyr Gln Gln Lys Thr Asn Glu Ser Pro
    50                  55                  60

Arg Leu Leu Ile Lys Tyr Ser Ser Gln Ser Ile Ser Gly Ile Pro Ser
65                  70                  75                  80

Arg Phe Ser Ala Ser Gly Ser Gly Thr Asp Phe Thr Leu Asn Ile Asn
                85                  90                  95

Asn Val Glu Phe Asp Asp Val Ser Ser Tyr Phe Cys Gln Gln Thr Gln
            100                 105                 110

Ser Trp Pro Asp Thr Phe Gly Ala Gly Thr Lys Leu Glu Leu Lys Arg
        115                 120                 125

Ala Asp Ala Ala Pro Thr Val Ser Ile Phe Pro Pro Ser Thr Glu Gln
    130                 135                 140

Leu Ala Thr Gly Gly Ala Ser Val Val Cys Leu Met Asn Asn Phe Tyr
145                 150                 155                 160

Pro Arg Asp Ile Ser Val Lys Trp Lys Ile Asp Gly Thr Glu Arg Arg
                165                 170                 175

Asp Gly Val Leu Asp Ser Val Thr Asp Gln Asp Ser Lys Asp Ser Thr
            180                 185                 190

Tyr Ser Met Ser Ser Thr Leu Ser Leu Thr Lys Ala Asp Tyr Glu Ser
        195                 200                 205

His Asn Leu Tyr Thr Cys Glu Val Val His Lys Thr Ser Ser Ser Pro
    210                 215                 220

Val Val Lys Ser Phe Asn Arg Asn Glu Cys
225                 230

<210>  33
<211>  106
<212>  PRT
<213>  Rattus norvegicus

<400>  33

Ala Asp Ala Ala Pro Thr Val Ser Ile Phe Pro Pro Ser Thr Glu Gln
1               5                   10                  15

Leu Ala Thr Gly Gly Ala Ser Val Val Cys Leu Met Asn Asn Phe Tyr
            20                  25                  30

Pro Arg Asp Ile Ser Val Lys Trp Lys Ile Asp Gly Thr Glu Arg Arg
        35                  40                  45

Asp Gly Val Leu Asp Ser Val Thr Asp Gln Asp Ser Lys Asp Ser Thr
    50                  55                  60

Tyr Ser Met Ser Ser Thr Leu Ser Leu Thr Lys Ala Asp Tyr Glu Ser
65                  70                  75                  80

His Asn Leu Tyr Thr Cys Glu Val Val His Lys Thr Ser Ser Ser Pro
                85                  90                  95

Val Val Lys Ser Phe Asn Arg Asn Glu Cys
            100                 105

## Description

### Technical Field

The present invention relates to a method for determining a nucleic acid sequence of a target gene. Priority is claimed on Provisional Application No. 62/302,196 provisionally filed to the United States on March 2, 2016, the content of which is incorporated herein by reference.

### Background Art

For example, hybridoma production technology is widely accepted as a means for preparing large amounts of monoclonal antibodies and for research and clinical applications. However, if culturing of hybridoma is continued, there is a concern that reactivity of a produced antibody is changed due to somatic mutations. For this reason, in a case of preserving a useful antibody and preparing a modified antibody, it is required to determine a nucleic acid sequence of a gene of the antibody produced by hybridoma.

In the related art, in determining a nucleic acid sequence of an antibody gene, the method of 5' Rapid Amplification of cDNA Ends (5' RACE), the degenerative PCR method, and the like can be used (for example, refer to NPL 1)

### Citation List

### Non-Patent Literature

[NPL 1] Zhou, H., et al., Optimization of primer sequences for mouse scFv repertoire display library construction., Nucleic Acids Research, 22 (5), 888-889, 1994.

### Summary of Invention

### Technical Problem

However, the 5' RACE method requires a large amount of total RNA and is difficult to carry out in some cases. In addition, in the degenerative PCR method, there are cases where loss of the original nucleic acid sequence is caused due to mis-hybridization of degenerative primers.

An object of the present invention is to provide a method for conveniently and accurately determining a nucleic acid sequence of a target gene expressed in a subject cell. Solution to Problem

The present invention includes the following aspects.
[1] A method for determining a nucleic acid sequence of a target gene expressed in a subject cell, including comprehensively determining mRNA nucleic acid sequences in the subject cell, and identifying a nucleic acid sequence having a nucleic acid sequence of a portion of the target gene, from among the determined mRNA nucleic acid sequences, in which the identified nucleic acid sequence is a nucleic acid sequence of the target gene.
[2] The method according to [1], in which a rank of the target gene is first to tenth in a case where the ranks of all genes expressed in the subject cell are determined in order from the largest number of mRNA molecules.
[3] The method according to [1] or [2], in which the subject cell is an antibody-producing cell, and in which the target gene is an antibody heavy chain gene and a nucleic acid sequence of a portion of the target gene is a nucleic acid sequence of a portion of a constant region of the antibody heavy chain gene, or the target gene is an antibody light chain gene and a nucleic acid sequence of a portion of the target gene is a nucleic acid sequence of a portion of a constant region of the antibody light chain gene.
[4] The method according to any one of [1] to [3], in which comprehensively determining mRNA nucleic acid sequences is performed by next generation sequencing.
[5] The method according to [4], in which the number of reads of the nucleic acid sequence in the next generation sequencing is 50,000 reads or less.

### Advantageous Effects of Invention

According to the present invention, it is possible to provide a new technology capable of conveniently and accurately determining a nucleic acid sequence of a target gene expressed in a subject cell.

### Brief Description of Drawings

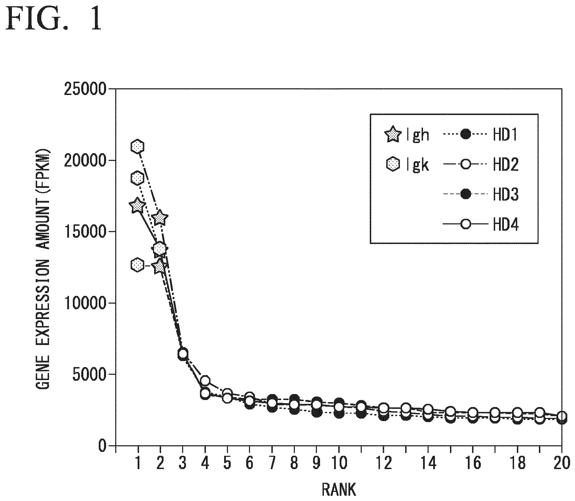

FIG. 1 is a graph in which transcriptomes are ordered in order from the highest expression level in Experimental Example 1.

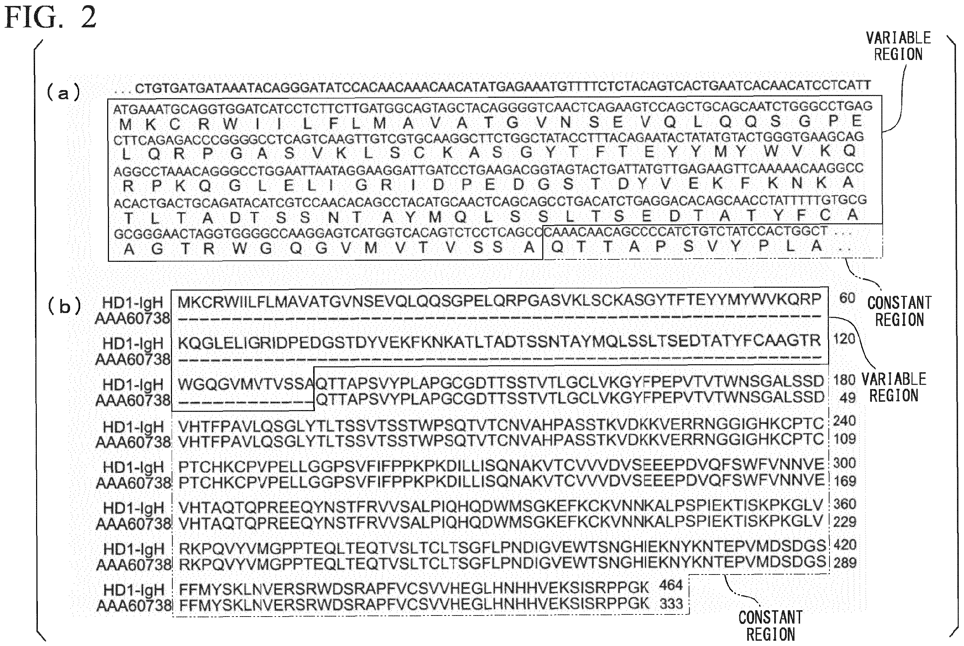

FIG. 2(a) is a figure showing a nucleic acid sequence (Sequence Number 28) of Igh of hybridoma clone HD1 and an estimated amino acid sequence (Sequence Number 29) in Experimental Example 2. FIG. 2(b) is a figure in which an amino acid sequence of IgH protein of clone HD1 of which a nucleic acid sequence is determined in Experimental Example 2, and an amino acid sequence (accession number: AAA6078, Sequence Number 30) of a constant region of the known rat IgH (IgG2b) are aligned.

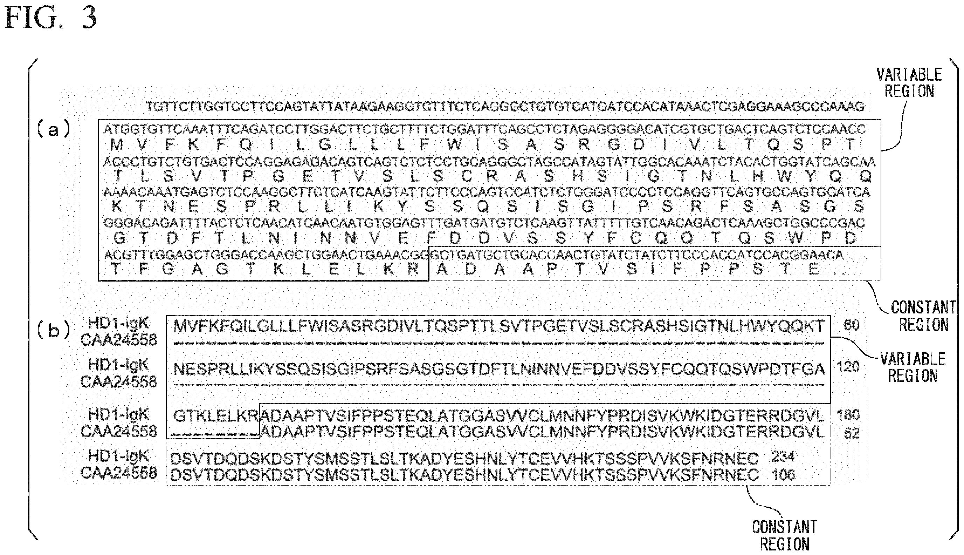

FIG. 3(a) is a figure showing a nucleic acid sequence (Sequence Number 31) of Igk of hybridoma clone HD1 and an estimated amino acid sequence (Sequence Number 32) in Experimental Example 2. FIG. 3(b) is a figure in which an amino acid sequence of IgK protein of clone HD1 of which a nucleic acid sequence is determined in Experimental Example 2 and an amino acid sequence (accession number: CAA24558, Sequence Number 33) of a constant region of the known rat IgK in Experimental Example 2 are aligned.

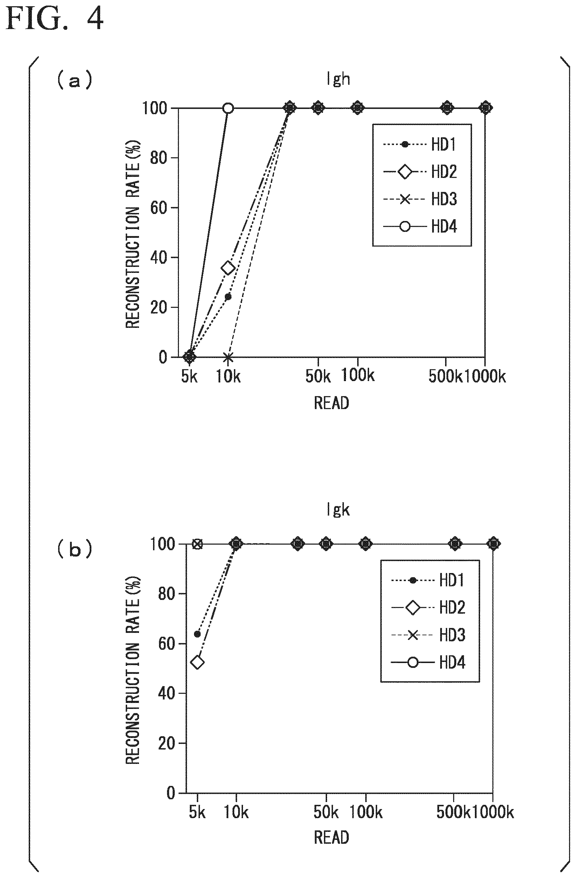

FIG. 4(a) is a graph showing a result obtained by calculating a reconstruction rate of Igh in a case of performing de novo assembly from reads of each read number in Experimental Example 4. FIG. 4(b) is a graph showing a result obtained by calculating a reconstruction rate of Igk in a case of performing de novo assembly from reads of each read number in Experimental Example 4.

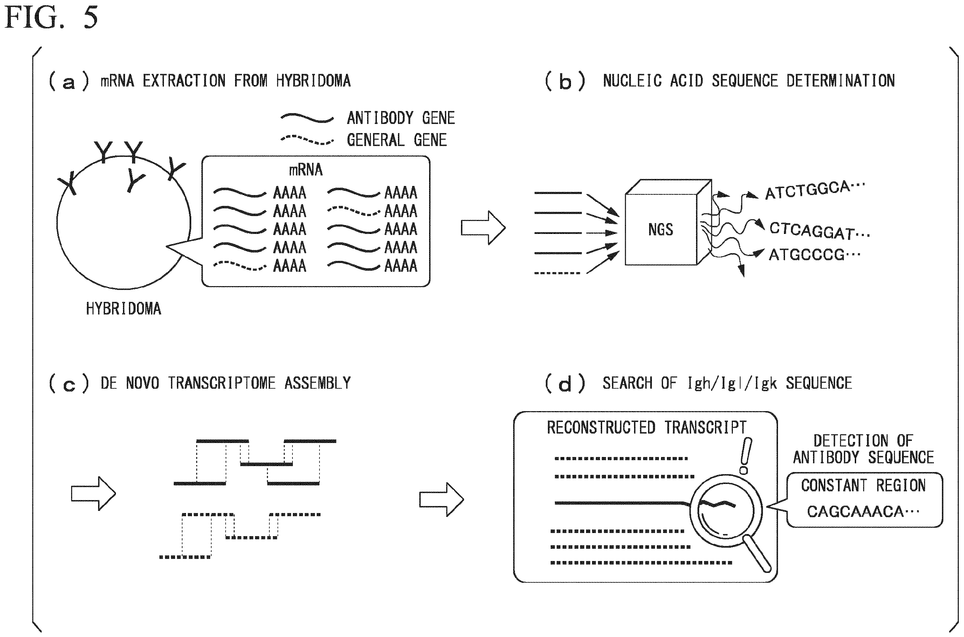

FIG. 5(a) to 5(d) are figures showing a method for determining a nucleic acid sequence according to an embodiment.

### Description of Embodiments

In Embodiment 1, the present invention provides a method for determining a nucleic acid sequence of a target gene expressed in a subject cell, including a step of comprehensively determining mRNA nucleic acid sequences in the subject cell and a step of identifying a nucleic acid sequence having a nucleic acid sequence of a portion of the target gene, from among the determined mRNA nucleic acid sequences, in which the identified nucleic acid sequence is a nucleic acid sequence of a target gene.

According to the method of the present embodiment, it is possible to conveniently and accurately determine a nucleic acid sequence of a target gene. In addition, the method of the present embodiment can be carried out using only approximately 0.1 µg of total RNA. For this reason, with one subject cell as a sample, for example, a nucleic acid sequence of a target gene at one cell level can be determined.

In the method of the present embodiment, the step of comprehensively determining mRNA nucleic acid sequences is preferably performed by next generation sequencing. More specifically, the method of the present embodiment can be carried out by mRNA-seq that comprehensively determines mRNA nucleic acid sequences by the next generation sequencing.

"Next generation sequencing (NGS)" is a term that is used in comparison with a first generation sequencer represented by a fluorescent capillary sequencer using sequencing method by the Sanger method. The next generation sequencing substantially includes various machines or technologies, and it is assumed that various forms of the next generation sequencers will be designed from now on.

In the first generation sequencer, the number of specimens that can be processed at a time is limited to a maximum of approximately 96. In addition, a DNA molecule used as a sample for performing sequence determination was required to be prepared by being separately cloned in advance and amplified by the PCR method, and enormous efforts were required at that stage.

In contrast, in the next generation sequencing using the next generation sequencer, DNA fragments including various sequences are arranged in parallel and analyzed by applying amplification technologies such as emulsion PCR and bridge PCR or high-sensitivity detection technology such as one molecule observation. For this reason, larger-scale nucleic acid sequences can be conveniently determined.

Specific examples of the next generation sequencer include MiSeq, HiSeq, NovaSeq (Illumina); Genetic Analyzer V2.0, Ion Proton (Thermo Fisher Scientific); MinION, PromethION (Nanopore), and the like.

In the step of comprehensively determining mRNA nucleic acid sequences in a subject cell, library preparation is performed depending on the type of the next generation sequencer that is used. For example, sequencing may be performed at an average read length of 50 to 100 bp and a number of reads of 30,000 to 50,000. That is, the number of reads of nucleic acid sequences by the next generation sequencing may be 50,000 reads or less. As will be described later in the examples, according to the method of the present embodiment, even if the number of reads is small to that extent, a nucleic acid sequence of a target gene can be determined.

Nucleic acid sequence data obtained by sequencing obtains contigs by assembling (attaching) due to an optional technique. In the specification of the present application, "contigs" refers to longer nucleic acid sequences obtained by assembling short reads. For example, contigs are the assembled full-length mRNA nucleic acid sequences. As a result, the mRNA nucleic acid sequence in the subject cell is determined. For example, assembling can be performed by a technique such as de novo assembly which does not require a reference sequence.

Subsequently, a nucleic acid sequence having a nucleic acid sequence of a portion of a target gene is identified from among the determined mRNA nucleic acid sequences (contigs). The nucleic acid sequence identified in this way is the nucleic acid sequence of the target gene.

For example, in a case where the target gene is an antibody gene, a nucleic acid sequence of a constant region of an antibody heavy chain (Igh) and a nucleic acid sequence of a constant region of an antibody λ light chain (Igl) or an antibody κ light chain (Igk) can be used as nucleic acid sequences of a portion of the target gene. More specifically, for example, nucleic acid sequences described in Sequence Numbers 11 to 14 can be used as nucleic acid sequences of a portion of a constant region of rat Igh. In addition, for example, nucleic acid sequences described in Sequence Numbers 15 and 16 can be used as nucleic acid sequences of a portion of the constant region of rat Igl. In addition, for example, a nucleic acid sequence described in Sequence Number 17 can be used as a nucleic acid sequence of a portion of a constant region of rat Igk. In addition, for example, nucleic acid sequences described in Sequence Numbers 18 to 22 can be used as nucleic acid sequences of a portion of a constant region of mouse Igh. In addition, for example, nucleic acid sequences described in Sequence Numbers 23 to 26 can be used as nucleic acid sequences of a portion of a constant region of mouse Igl. In addition, for example, a nucleic acid sequence described in Sequence Number 27 can be used as a nucleic acid sequence of a portion of a constant region of mouse Igk.

In addition, it is possible to extract a nucleic acid sequence of the target gene more efficiently by identifying a contig having a nucleic acid sequence of a portion of the target gene and having a total length equal to or greater than the total length of the target gene.

For example, in a case where the target gene is Igh, an Igh amino acid sequence includes 400 or more amino acid residues. Here, it may be possible to identify a contig having a nucleic acid sequence having a length of 1200 bp or greater required for coding the amino acid sequence. In this way, it is possible to efficiently extract the contig of a full-length target gene.

In the method of the present embodiment, the target gene that determines a nucleic acid sequence is preferably a gene of which a rank is first to tenth in a case where the ranks of all genes expressed in the subject cell are determined in order from the largest number of mRNA molecules. It is possible to easily determine a nucleic acid sequence of the target gene having a large number of mRNA molecules and within the above range.

Or, the expression amount of the target gene is preferably 5,000 fragments per kilobase of exon per million mapped fragments (FPKM) or greater. It is possible to easily determine the nucleic acid sequence of the target gene of which an expression amount is at that degree. The upper limit of the expression amount of the target gene is not particularly limited, but in general, 30,000 FPKM or so is the upper limit in many cases.

Examples of the target gene include an antibody gene, a T-cell receptor gene, a B-cell receptor gene, and the like, but the target gene is not limited thereto.

### (Antibody gene)

For example, the subject cell may be an antibody-producing cell, the target gene may be an antibody heavy chain gene, and a nucleic acid sequence of a portion of the target gene may be a nucleic acid sequence of a portion of a constant region of the antibody heavy chain gene. Or, the subject cell may be an antibody-producing cell, the target gene may be an antibody light chain gene, and a nucleic acid sequence of a portion of the target gene may be a nucleic acid sequence of a portion of a constant region of the antibody light chain gene.

In recent years, for example, there have been cases where an antibody was produced by using an animal of which a genome nucleic acid sequence was not clear, such as skunk. In such a case, there are cases where a genome nucleic acid sequence cannot be used in a reference sequence of determination of a nucleic acid sequence. According to the method of the present embodiment, since it is possible to determine a nucleic acid sequence even in a case where a reference sequence is not present, it is possible to determine a nucleic acid sequence of an antibody gene even in such a case.

In addition, in the conventional method for determining a nucleic acid sequence of an antibody gene, it was not possible to identify only a nucleic acid sequence of a variable region. In contrast, according to the method of the present embodiment, it is possible to determine a total length of a nucleic acid sequence of the target gene also including a constant region. For this reason, as will be described later in the examples, in a case where the target gene is an antibody gene, it is possible to identify even isotypes or subclasses of an antibody. In addition, for example, it is also possible to detect a small number of mutants due to somatic mutation of an antibody gene.

### (T-cell receptor gene)

For example, in adoptive immunotherapy for cancer and the like, there is a demand for determining a nucleic acid sequence of a T-cell receptor. Here, for example, the subject cell may be a T cell, the target gene may be a T-cell receptor gene, and a nucleic acid sequence of a portion of the target gene may be a nucleic acid sequence of a portion of a constant region of a T-cell receptor gene.

### (B-cell receptor gene)

For example, the subject cell may be an immature B cell, the target gene may be a B-cell receptor heavy chain gene, and a nucleic acid sequence of a portion of the target gene may be a nucleic acid sequence of a portion of a constant region of a B-cell receptor heavy chain gene. Or, the subject cell may be an immature B cell, the target gene may be a B-cell receptor light chain gene, and a nucleic acid sequence of a portion of the target gene may be a nucleic acid sequence of a portion of a constant region of a B-cell receptor light chain gene.

### (Other target genes)

The target gene is not limited to the above genes, and may be an optional gene. According to the method of the present embodiment, for example, regarding the optional target gene, single nucleotide variants (SNVs), single nucleotide polymorphysms (SNPs), insertion/deletion (Indel), splicing variants, and the like can be easily analyzed.

### Examples

Next, the present invention will be described in detail by showing examples, but the present invention is not limited to the following examples.

### [Methods and materials]

### (Cell lines)

Hybridoma cell lines (clones HD1, HD2, HD3, and HD4) established by the inventors were used in experiments. Each hybridoma was cultured by using a hybridoma serum-free culture medium (Gibco) to which 10% fetal bovine serum (FBS) was added, 1.2% penicillin-streptomycin-glutamine (Gibco), 1 ng/mL interleukin (IL)-6 or a GIT culture medium (Waco Pure Chemical Industries) to which 1 ng/mL IL-6 was added.

### (mRNA-seq)

From each hybridoma cell line, total RNA was prepared by using a commercially available kit (form "AllPrep DNA/RNA Mini Kit", QIAGEN). A library was prepared by using 1 µg of total RNA and a commercially available kit (form "NEBNext Ultra Directional RNA Library Prep Kit", New England Biolabs). With the kit, it is possible to produce a library by decreasing the used total RNA to approximately 0.1 µg.

Subsequently, mRNA-seq was performed by paired-end sequencing at an average read length of 50 bp using a next generation sequencer (form "HiSeq 1500", Illumina). For each hybridoma cell line, nucleic acid sequence data having the number of reads of 40 × 10⁶ reads or more was obtained.

### (mRNA-seq data analysis)

The obtained reads were mapped against the inventors' custom transcriptome reference sequence. The custom transcriptome reference sequence included mouse transcripts, rat transcripts, rat immunoglobulin heavy chain (Igh) constant region, immunoglobulin λ light chain (Igl) constant region, and immunoglobulin κ light chain (Igk) constant region nucleic acid sequences.

The reads were mapped with the parameter of -t8-P-L 10000 by using BWA-MEM which is a mapping program. The program TIGAR2 was used with default settings. The expression level of each gene was quantified as fragments per kilobase of exon per million mapped fragments (FPKM).

### (De novo transcriptome assembly)

Total reads or subsampled reads by the program "fastq-sample" (http://homes.cs.washington.edu/∼dcjones/fastq-tools) were de novo assembled using the program "Trinity". CPU and max-memory parameters were changed according to read numbers. For example, in a case where the number of the reads was 40 × 10⁶ reads, the CPU parameter was set to 8, and the max-memory parameter was set to 52G. In addition, for example, in a case where the number of the reads was 1 × 10⁶ reads, the CPU parameter was set to 2, and the max-memory parameter was set to 12G.

The Igh and Igl/Igk coding nucleic acid sequences (CDS) were extracted by filtering treatment in a case where the contigs (assembled nucleic acid sequences) contained 20-to-30-bp unique nucleic acid sequences of the Igh and Igl/Igk constant region and had proper length (in the case of Igh, more than 1200 bp, and in the case of Igl/Igk, more than 600 bp).

### (RT-PCR)

Each hybridoma RNA was purified by phenol/chloroform extraction. Reverse transcription reaction was performed by using a commercially available kit (form "PrimeScript (trademark) II 1st strand cDNA Synthesis Kit", TAKARA BIO INC.). PCR was performed using an enzyme (form "KOD Plus", TOYOBO) and a thermal cycler. PCR products were purified by gel extraction to remove non-specific products. After that, nucleic acid sequences were determined by the Sanger method. Sequence numbers of nucleic acid sequences of primers using PCR are shown in Table 1.

**[Table 1]**

| | Sense primer (Sequence Number) | Antisense primer (Sequence number) |
|---|---|---|
| HD1-Igh | 1 | 5 |
| HD2-Igh | 2 | 5 |
| HD3-Igh | 3 | 5 |
| HD4-Igh | 4 | 5 |
| HD1-Igk | 6 | 10 |
| HD2-Igk | 7 | 10 |
| HD3-Igk | 8 | 10 |
| HD4-Igk | 9 | 10 |

### [Experimental Example 1]

### (mRNA-seq analysis of hybridoma)

Clones HD1, HD2, HD3, and HD4 which are hybridoma cell lines established as fusion cells of rat B lymphocytes and mouse myeloma cell line SP2 were subjected to mRNA-seq. Paired-end sequencing was performed at an average read length of 50 bp.

Subsequently, each transcriptome expression level was quantified by the program BWA-TIGAR2 and ordered according to expression levels. FIG. 1 is a graph in which transcriptomes are ordered in order from the highest expression level.

As a result, it was clarified that in all the hybridoma clones, the Igh and Igl/Igk coding nucleic acid sequences had an expression amount of more than 10,000 FPKM, and were ranked as the transcript having the highest expression level.

The result shows that the mRNA-seq data of hybridomas contains a sufficient number of reads to reconstruct the Igh and Igl/Igk coding nucleic acid sequences.

### [Experimental Example 2]

### (Assembly of Igh and Igl/Igk nucleic acid sequences of rat hybridoma)

Reconstruction of the Igh and Igl/Igk nucleic acid sequences was attempted by de novo transcriptome assembly of the mRNA-seq data obtained in Experimental Example 1.

First, reconstruction of a full-length transcriptome was performed from the mRNA-seq data of clone HD1 using the program Trinity. Here, filtering of the reads was not performed. The number of reads was 45,406,048 reads, and the number of obtained contigs was 58,822 contigs.

Subsequently, the Igh coding nucleic acid sequence was extracted by filtering. In the filtering, contigs having 20-to-30-bp unique nucleic acid sequences of the Igh constant region were extracted. Sequence Numbers of the nucleic acid sequences used in the filtering are shown in the following Table 2.

**[Table 2]**

| | Sequence Number |
|---|---|
| rat-Ighg1 | 11 |
| rat-Ighg2a | 12 |
| rat-Ighg2b | 13 |
| rat-Ighg2c | 14 |

The full-length IgH contains 400 or more residues of amino acids. Here, a 1395-bp nucleic acid sequence was identified as the Igh nucleic acid sequence having a length of 1,200 bp or more and containing the unique 24-bp nucleic acid sequence of Ighg2b. The identified Igh nucleic acid sequence was identical to the Igh nucleic acid sequence of clone HD1 of which the nucleic acid sequence was determined by the Sanger method.

FIG. 2(a) is a figure showing the Igh nucleic acid sequence (Sequence Number 28) and an estimated amino acid sequence (Sequence Number 29) of clone HD1 of which the nucleic acid sequence was determined by de novo transcriptome assembly. FIG. 2(b) is a figure in which an amino acid sequence of IgH protein of clone HD1 of which a nucleic acid sequence was determined by de novo transcriptome assembly, and an amino acid sequence (accession number: AAA6078, Sequence Number 30) of the constant region of the known rat IgH (IgG2b) are aligned. As a result, it was clarified that NOS. 133 to 464 from among amino acid sequences of IgH produced by clone HD1 are matched with the amino acid sequences of the constant region of the known rat IgH.

Subsequently, the Igl/Igk coding nucleic acid sequences were extracted by filtering. In the filtering, the contigs having 20-to-30-bp unique nucleic acid sequences of the Igl/Igk constant region were extracted. Sequence Numbers of the nucleic acid sequences used in the filtering are shown in the following Table 3.

**[Table 3]**

| | Sequence Number |
|---|---|
| rat-Igl1 | 15 |
| rat-Igl2 | 16 |
| rat-Igk | 17 |

The full-length IgK had 200 or more residues of amino acids. Here, a 705-bp nucleic acid sequence was identified as an Igk nucleic acid sequence having a length of 600 bp or more and containing a unique Igk nucleic acid sequence. The identified Igk nucleic acid sequence was identical to the Igk nucleic acid sequence of clone HD1 of which the nucleic acid sequence was determined by the Sanger method.

FIG. 3(a) is a figure showing an Igk nucleic acid sequence (Sequence Number 31) of clone HD1 of which a nucleic acid sequence was determined by de novo transcriptome assembly and an estimated amino acid sequence (Sequence Number 32). FIG. 3(b) is a figure in which an amino acid sequence of IgK protein of clone HD1 of which a nucleic acid sequence is determined by de novo transcriptome assembly and an amino acid sequence (accession number: CAA24558, Sequence Number 33) of a constant region of the known rat IgK are aligned. As a result, it was clarified that NOS. 129 to 234 from among amino acid sequences of IgK produced by clone HD1 are matched with the amino acid sequences of the constant region of the known rat IgK.

At the same time, the inventors also identified nucleic acid sequences of antibody genes produced by clone HD2 (Ighg2a/Igk), clone HD3 (Ighg2a/Igk), and clone HD4 (Ighg2a/Igk).

In addition, it was confirmed that the antibody isotypes produced by clones HD1 to HD4 are matched with the results of isotyping assay by ELISA. The above result shows that nucleic acid sequences of the Igh genes and Igl/Igk genes can be conveniently and accurately determined by de novo assembly of mRNA-seq data.

### [Experimental Example 3]

### (Assembly of Igh and Igl/Igk nucleic acid sequence of mouse hybridoma)

Similar to Experimental Example 2, Igh and Igk nucleic acid sequences of clone 8A2 and 13C7 which are mouse hybridomas were determined. Sequence numbers of the nucleic acid sequence used in filtering of mouse Igh and Igl/Igk are shown in the following Table 4.

**[Table 4]**

| | Sequence Number |
|---|---|
| mouse-Ighg1 | 18 |
| mouse- Ighg2a | 19 |
| mouse- Ighg2b | 20 |
| mouse-Ighg2c | 21 |
| mouse-Ighg3 | 22 |
| mouse-Igl1 | 23 |
| mouse-Igl2 | 24 |
| mouse-Igl3 | 25 |
| mouse-Igl4 | 26 |
| mouse-Igk | 27 |

As a result, it was confirmed that the nucleic acid sequences are matched with nucleic acid sequences of these monoclonal antibodies determined by the Sanger method, except for a region coded by degenerative primers. The result is further evidence that nucleic acid sequences of the Igh genes and the Igl/Igk genes can be conveniently and accurately determined by de novo assembly of mRNA-seq data. In addition, it was confirmed that in the method using degenerative primers, there are cases where loss of the original nucleic acid sequence occurs due to mis-hybridization of degenerative primers.

### [Experimental Example 4]

### (Optimization of de novo assembly conditions for determining nucleic acid sequence of antibody gene)

Optimization of conditions for determining nucleic acid sequences of the Igh and Igl/Igk genes was attempted using hybridoma mRNA-seq data. First, the required number of reads for determining nucleic acid sequences of antibody genes was examined.

More specifically, among total reads of mRNA-seq of each of clones HD1 to HD4, 5 × 10³ reads, 10 × 10³ reads, 30 × 10³ reads, 50 × 10³ reads, 100 × 10³ reads, 500 × 10³ reads, and 1000 × 10³ reads were randomly subsampled.

Subsequently, de novo assembly was repeatedly carried out 25 times using the reads. Subsequently, the Igh and the Igl/Igk nucleic acid sequences identified using total reads were defined as correct nucleic acid sequences, and the success rate (reconstruction rate) of obtaining complete nucleic acid sequences was calculated.

FIG. 4(a) is a graph showing a result obtained by calculating the reconstruction rate of Igh in a case of performing de novo assembly from reads of each read number. As a result, it was clarified that in all four clones, the Igh nucleic acid sequence can be completely identified with more than 30 × 10³ reads. FIG. 4(b) is a graph showing a result obtained by calculating a reconstruction rate of Igk in a case of performing de novo assembly from reads of each read number. As a result, it was clarified that in all four clones, the Igk nucleic acid sequence can be completely identified with more than 10 × 10³ reads.

The result shows that this method can accurately identify nucleic acid sequences of antibody genes from mRNA-seq data of a limited number of reads. FIG. 5 is a figure putting together a method for determining a nucleic acid sequence according to this method. First, as shown in FIGS. 5(a) and 5(b), mRNA is extracted from cells and nucleic acid sequences are determined by next generation sequencing (NGS). Subsequently, as shown in FIG. 5(c), data obtained by mRNA-seq is de novo assembled to prepare contigs. Subsequently, as shown in FIG. 5(d), a contig having a specific nucleic acid sequence is identified by filtering to obtain a nucleic acid sequence of a target gene.

### [Experimental Example 5]

### (Determination of nucleic acid sequence of antibody gene produced by other hybridomas)

This method for determining a nucleic acid sequence was applied to a greater numbers of hybridomas to determine nucleic acid sequences of antibody genes. As a result, in 96 or more kinds of hybridomas, it was possible to perform mRNA-seq with 200 × 10³ reads per sample to accurately determine nucleic acid sequences of antibody genes.

### Industrial Applicability

According to the present invention, it is possible to provide a new technology capable of conveniently and accurately determining a nucleic acid sequence of a target gene expressed in a subject cell.

## Claims

1. A method for determining a nucleic acid sequence of a target gene expressed in a subject cell, the method comprising:
comprehensively determining mRNA nucleic acid sequences in the subject cell, and
identifying a nucleic acid sequence having a nucleic acid sequence of a portion of the target gene, from among the determined mRNA nucleic acid sequences,
wherein the identified nucleic acid sequence is a nucleic acid sequence of the target gene.

2. The method according to Claim 1,
wherein a rank of the target gene is first to tenth in a case where the ranks of all genes expressed in the subject cell are determined in order from the largest number of mRNA molecules.

3. The method according to Claim 1 or 2,
wherein the subject cell is an antibody-producing cell, and
wherein the target gene is an antibody heavy chain gene and a nucleic acid sequence of a portion of the target gene is a nucleic acid sequence of a portion of a constant region of the antibody heavy chain gene, or the target gene is an antibody light chain gene and a nucleic acid sequence of a portion of the target gene is a nucleic acid sequence of a portion of a constant region of the antibody light chain gene.

4. The method according to any one of Claims 1 to 3,
wherein comprehensively determining mRNA nucleic acid sequences is performed by next generation sequencing.

5. The method according to Claim 4,
wherein the number of reads of the nucleic acid sequence in the next generation sequencing is 50,000 reads or less.
